# EUROPÄISCHE PATENTSCHRIFT

(11) **EP 3 254 608 B1**
(45) Veröffentlichungstag und Bekanntmachung des Hinweises auf die Patenterteilung: **02.03.2022**
(21) Anmeldenummer: 17171299.5
(22) Anmeldetag: 16.05.2017
(51) Int. Cl.: A61B 1/00, A61B 1/002, G02B 23/24

(54) **ENDOSKOP UND VERFAHREN ZUR HERSTELLUNG EINES ENDOSKOPS**
ENDOSCOPE AND METHOD FOR MANUFACTURING AN ENDOSCOPE
ENDOSCOPE ET SON PROCÉDÉ DE FABRICATION

(30) Priorität: 01.06.2016 DE 102016110114
(43) Veröffentlichungstag der Anmeldung: 13.12.2017
(73) Patentinhaber: Karl Storz SE & Co. KG, 78532 Tuttlingen (DE)
(72) Erfinder: Eisenlauer, Johannes, 78532 Tuttlingen (DE); Deutschendorf, Andreas, 78532 Tuttlingen (DE)

(56) Entgegenhaltungen:
- EP-A1- 2 453 288
- DE-U- 1 977 884
- US-A- 4 148 551
- US-A1- 2014 066 714

## Beschreibung

Die vorliegende Erfindung betrifft ein Endoskop nach dem Oberbegriff von Anspruch 1 sowie ein Verfahren zur Herstellung eines Endoskops.

Endoskope werden heute für vielerlei Anwendungen in Medizin und Technik verwendet. Ein Endoskop umfasst typischerweise einen langerstreckten Schaft und einen am proximalen (d.h. beobachternahen) Ende des Schafts angeordneten Endoskopkopf. Der Schaft kann insbesondere starr ausgebildet sein und ein starres Außenrohr aufweisen. Im distalen (beobachterfernen) Endbereich des Schafts ist zur Erzeugung eines Bildes eines Objektfelds in dem Hohlraum ein Endoskopobjektiv angeordnet. Das endoskopische Bild kann über einen im Innern des Schafts angeordneten Bildweiterleiter zum Endoskopkopf weitergeleitet werden, wo es beispielsweise zur Betrachtung durch den Beobachter zur Verfügung steht. Ferner kann innerhalb des Schafts ein Beleuchtungslichtleiter angeordnet sein, um Beleuchtungslicht an das distale Ende des Endoskops zu transportieren. Ein derartiges Endoskop wird auch als "Endoskopoptik" bezeichnet.

Der Bildweiterleiter, der beispielsweise durch eine Anzahl von aufeinander folgenden Stablinsen gebildet werden kann, ist in der Regel in einem langerstreckten Rohr aufgenommen, das auch als "Systemrohr" oder "Optikrohr" bezeichnet wird und das bei der Herstellung des Endoskops in das Außenrohr oder in ein innerhalb des Außenrohrs angeordnetes Innenrohr des Schafts eingeschoben wird. Nach dem Einschieben wird das Systemrohr in axialer Richtung innerhalb des Endoskops fixiert, beispielsweise mit Madenschrauben oder durch Verschweißen. Dies ist mit einem relativ hohen Zeitaufwand verbunden; zudem ist es nicht immer möglich, das Systemrohr etwa zu Reparaturzwecken wieder zu entnehmen.

Aus EP 2 453 288 A1 ist es bekannt, dass eine Objektivfassung durch eine Druckfeder in distaler Richtung belastet wird, wobei ein Widerlager für die Druckfeder als geschlitzte federelastische Hülse ausgebildet ist. Der distalseitige Rand der Hülse weist einen umlaufenden Rastvorsprung auf, der im montierten Zustand in eine umlaufende Rastnut im Endoskopgehäuse eingreift. Durch die Druckfeder wird die Objektivfassung in distaler Richtung gegen einen Absatz im Endoskopgehäuse gedrückt, um das Objektiv in axialer Richtung positionsstabil im Endoskopgehäuse zu positionieren. Die Hülse ist sich in proximaler Richtung konisch verjüngend ausgebildet. Zum Lösen der Rastverbindung kann zwischen Hülse und Endoskopgehäuse ein Demontagerohr auf die konische Außenwand der Hülse aufgeschoben werden. Ein Systemrohr mit einem Bildweiterleiter wird hierdurch nicht fixiert. Die konisch ausgebildete, federelastische Hülse sowie der zum Einführen des Demontagerohrs notwendige Zwischenraum nehmen einen Teil des verfügbaren Lumens im distalen Endbereich des Schafts ein, so dass der für den Bildweiterleiter zur Verfügung stehende Raum eingeschränkt wird, was insbesondere bei dünnen Endoskopen nachteilig

Ein Endoskop nach der Präambel von Anspruch 1 ist aus EP2453288 A1 bekannt.

Es ist Aufgabe der vorliegenden Erfindung, ein Endoskop und ein Verfahren zur Herstellung eines Endoskops anzugeben, wobei die oben genannten Nachteile möglichst vermieden werden und wobei insbesondere eine vereinfachte Montage mit einer einfachen axialen Fixierung des Systemrohrs ermöglicht wird.

Diese Aufgabe wird durch ein Endoskop gemäß Anspruch 1, durch ein Verfahren gemäß Anspruch 11 gelöst.

Vorteilhafte Weiterbildungen der Erfindung ergeben sich aus den Unteransprüchen.

Ein erfindungsgemäßes Endoskop, das auch als Endoskopoptik bezeichnet werden kann, weist einen langerstreckten Schaft auf, der insbesondere zur Einführung in einen körperinneren Hohlraum eines menschlichen oder tierischen Körpers oder auch in einen Hohlraum eines technischen Gegenstands geeignet ist und der vorzugsweise starr ausgebildet ist. In einem distalen Endbereich des Schafts ist in der Regel ein Objektiv zum Erzeugen eines Bilds eines Objektfelds, beispielsweise eines körperinneren Hohlraums, angeordnet. Weiter kann das Endoskop einen am proximalen Ende des Schafts angeordneten Endoskopkopf aufweisen. Das Endoskop kann hermetisch abgedichtet und sterilisierbar sein, vorzugsweise autoklavierbar.

Ferner weist das Endoskop ein Systemrohr auf, das zumindest mit einem Teil seiner Länge innerhalb des Schafts verläuft, insbesondere innerhalb eines Außenrohrs des Schafts. Das Systemrohr kann direkt in dem Außenrohr geführt sein oder beispielsweise mittels eines oder mehrerer in dem Außenrohr angeordneter weiterer Bauelemente des Endoskops. Innerhalb des Systemrohrs ist ein Bildweiterleiter angeordnet, der insbesondere durch eine Stablinsenanordnung, etwa durch ein oder mehrere Relaislinsensysteme, oder durch ein geordnetes Bündel von Lichtleitfasern gebildet wird. Der Bildweiterleiter dient zur Weiterleitung eines im distalen Endbereich des Schafts aufgenommenen endoskopischen Bilds, etwa des von dem Objektiv entworfenen Bilds des Objektfelds des körperinneren Hohlraums, in proximaler Richtung, wo das Bild betrachtet oder in anderer Weise ausgewertet werden kann. Der Bildweiterleiter kann sich insbesondere in proximaler Richtung über das proximale Ende des Systemrohrs hinaus erstrecken.

Erfindungsgemäß ist das Systemrohr mittels mindestens eines federnden Rastelements in einer axialen Richtung des Schafts relativ zum Schaft gehalten. Das federnde Rastelement kann hierfür ein oder mehrere federelastische Rastabschnitte aufweisen oder selbst insgesamt federelastisch ausgebildet sein. Das Rastelement schafft eine, ggf. über weitere Bauelemente vermittelte, Rastverbindung zwischen dem Systemrohr und dem Schaft. Vorzugsweise ist das Systemrohr durch das federnde Rastelement zumindest gegen eine Verschiebung in einer proximalen Richtung gehalten. In radialer Richtung kann das Systemrohr auf andere Weise gehalten bzw. festgelegt sein, beispielsweise durch eine Passung mit enger Toleranz. Eine Fixierung hinsichtlich einer Rotation des Systemrohrs um seine Längsachse kann ebenfalls vorgesehen sein.

Dadurch, dass das Systemrohr mittels mindestens eines federnden Rastelements in einer axialen Richtung des Schafts in dem Endoskop gehalten ist, wird es auf einfache Weise ermöglicht, das Systemrohr in einer axialen Richtung an einer vorgegebenen Position, die beispielsweise durch die Funktion des optischen Systems des Endoskops bestimmt sein kann, festzulegen. Dabei ermöglicht die Rastverbindung mittels eines federnden Rastelements eine besonders einfache Montage, bei der die Fixierung des Systemrohrs in axialer Richtung durch das federnde Einrasten des Rastelements, die die Rastverbindung zwischen dem Systemrohr und dem Schaft erzeugt, bewirkt werden kann. Insbesondere kann es ermöglicht werden, das Systemrohr in einem einzigen Arbeitsschritt oder in einer verringerten Anzahl von Arbeitsschritten in das Endoskopgehäuse einzusetzen und dort durch das Einrasten des Rastelements in axialer Richtung festzulegen. Eine aufwändige Fixierung mittels Schrauben oder Schweißen kann somit in der Regel entfallen.

Gemäß der Erfindung bildet das federnde Rastelement ein Widerlager für ein Federelement, das das Systemrohr in einer distalen axialen Richtung gegen einen Anschlag belastet. Dabei kann das Federelement direkt am Systemrohr angreifen oder dieses über ein oder mehrere mit dem Systemrohr verbundene Bauelemente, etwa über ein fest mit dem Systemrohr verbundenes Kopplungselement, in distaler Richtung belasten. Das Systemrohr wird durch das Federelement gegen den Anschlag gedrückt, der die Bewegung des Systemrohrs in distaler Richtung begrenzt. Das Federelement kann beispielsweise als Druckfeder ausgebildet sein, die sich proximalseitig am Rastelement und distalseitig am Systemrohr oder an dem mit dem Systemrohr verbundenen Kopplungselement abstützt. Dadurch, dass das Systemrohr durch das sich am Rastelement abstützende Federelement mit einer Federkraft gegen den Anschlag vorgespannt wird, kann auf einfache und sichere Weise eine Begrenzung der Einschubtiefe beim Einschieben des Systemrohrs in den Schaft ermöglicht und zugleich eine spielfreie Fixierung des Systemrohrs in axialer Richtung ermöglicht werden, wobei die in axialer Richtung wirkende Kraft durch die Auslegung und Kompression des Federelements vorbestimmt werden kann.

Vorzugsweise ist der Anschlag, gegen den das Systemrohr in distaler Richtung belastet ist, im distalen Endbereich des Schafts ausgebildet. Das Systemrohr ist relativ zum Schaft in der distalen Richtung vorgespannt und wird somit relativ zum distalen Endbereich des Schafts festgelegt. Hierdurch kann es sichergestellt werden, dass das Systemrohr und damit der in diesem angeordnete Bildweiterleiter im distalen Endbereich eine vorbestimmte axiale Position einnimmt, die beispielsweise in Beziehung zu Bauelementen des Objektivs definiert sein kann. Hierdurch ist eine besonders wirksame Fixierung des Bildweiterleiters und damit eine verbesserte Qualität des weitergeleiteten Bild erreichbar.

In besonders bevorzugter Weise wird der Anschlag, gegen den das Systemrohr in distaler Richtung vorgespannt ist, durch ein Deckglas gebildet, das im distalen Endbereich des Schafts befestigt ist. Das Systemrohr liegt insbesondere über ein Objektiv an dem Deckglas an, wobei das Objektiv zwischen dem Deckglas und dem Bildweiterleiter angeordnet ist. Das Objektiv kann teilweise mit dem Deckglas und/oder teilweise mit dem Systemrohr verbunden sein. Insbesondere kann eine Objektivhülse, in der ein oder mehrere optische Bauelemente des Objektivs aufgenommen sind, fest mit dem Systemrohr verbunden sein und dieses in distaler Richtung fortsetzen; ein oder mehrere weitere optische Bauelemente können an dem Deckglas anliegen oder sich direkt oder indirekt daran abstützen. Das Deckglas, das beispielsweise als planparallele Platte ausgebildet sein kann, dient insbesondere zum distalseitigen dichten Abschluss des Schafts und kann dicht in das distale Ende eines Außenrohrs oder eines Innenrohrs des Schafts eingesetzt sein. Hierdurch werden die Montage des Endoskops und die axiale Festlegung des Systemrohrs weiter vereinfacht.

Gemäß einer besonders bevorzugten Ausführungsform der Erfindung weist das Endoskop einen Endoskopkopf auf, der an einem proximalen Ende des Schafts angeordnet ist. Der Endoskopkopf ist in der Regel nicht zur Einführung in den Hohlraum vorgesehen und kann in radialer Richtung über die Außenkontur des Schafts bzw. über das Außenrohr des Schafts vorstehen. Im Endoskopkopf kann eine Bildbetrachtungseinrichtung vorgesehen sein, etwa ein Okular zum Betrachten des weitergeleiteten Bildes. Der Endoskopkopf weist ein Gehäuse auf, das mit dem proximalen Ende des Schafts, etwa mit einem Außenrohr des Schafts, vorzugsweise fest verbunden ist. Das Systemrohr erstreckt sich gemäß dieser Ausführungsform bis in den Endoskopkopf hinein, und das federnde Rastelement ist innerhalb des Endoskopkopfs angeordnet und verbindet einen proximalen Endbereich des Systemrohrs mit dem Gehäuse des Endoskopkopfs. Die vom Rastelement geschaffene Rastverbindung besteht insbesondere zwischen dem proximalen Endbereich des Systemrohrs oder einem mit diesem fest verbundenen Kopplungselement und dem Gehäuse oder einem mit diesem fest verbundenen Bauelement. Hierdurch kann in vorteilhafter Weise ausgenutzt werden, dass der Endoskopkopf radial gegenüber dem Schaft vorstehen kann und dass somit im Endoskopkopf mehr Raum für das federnde Rastelement als innerhalb des Außenrohrs des Schafts zur Verfügung steht. Da der Außendurchmesser des Schafts in der Regel durch die vorgesehene Verwendung des Endoskops vorgegeben ist, wird es hierdurch ermöglicht, das Systemrohr innerhalb des Endoskops mittels des Rastelements zu fixieren, ohne dass der Außendurchmesser des Systemrohrs oder der für den Bildweiterleiter zur Verfügung stehende Querschnitt verringert werden müsste.

Vorzugsweise ist das federnde Rastelement in mindestens eine Rastausnehmung auf einer Innenseite einer Innenhülse des Endoskopkopfs eingerastet, die fest mit dem Gehäuse des Endoskopkopfs verbunden ist. Hierfür können beispielsweise Rastabschnitte des Rastelements oder auch das Rastelement insgesamt in die mindestens eine Rastausnehmung durch Federkraft eingerückt sein und dadurch die Rastverbindung zwischen dem Systemrohr und dem Endoskopkopf schaffen. Die Rastausnehmung kann beispielsweise als in die Innenseite der Innenhülse eingebrachter Einstich, insbesondere als zumindest abschnittsweise umlaufende, ggf. unterbrochene Nut ausgebildet sein. Das Rastelement kann in einen Zwischenraum zwischen der Innenhülse und dem Kopplungselement eingesetzt sein. Hierdurch wird eine einfache, sichere und raumsparende Fixierung des Systemrohrs im Endoskop ermöglicht.

Vorzugsweise ist das Systemrohr mittels des federnden Rastelements lösbar gehalten. Insbesondere ist das Systemrohr mittels des Rastelements lösbar im Endoskop gehalten und kann nach Lösen der Rastverbindung entnommen werden, beispielsweise aus einem Außenrohr des Schafts sowie aus dem Endoskopkopf in proximaler Richtung herausgezogen werden. Hierfür kann es insbesondere vorgesehen sein, dass das Rastelement durch elastisches Verformen aus seiner Rastposition ausgerastet und in proximaler Richtung entnommen werden kann. Zu diesem Zweck kann ein entsprechend ausgebildetes Werkzeug erforderlich sein. Auf diese Weise kann ein Ersetzen oder eine Reparatur des Systemrohrs oder der in diesem aufgenommenen optischen Elemente erleichtert werden.

Vorzugsweise ist das Rastelement einstückig ausgebildet. Das federnde Rastelement ist insbesondere als Kunststoffclip ausgebildet und aus einem Kunststoffmaterial gefertigt, beispielsweise aus Polyetheretherketon (PEEK); das federnde Rastelement kann aber auch beispielsweise aus einem geeigneten metallischen Werkstoff bestehen, etwa aus Titan. Hierdurch kann die Herstellung vereinfacht werden und es zudem erreicht werden, dass das Rastelement dauerhaft federelastisch bleibt und den auf Endoskope etwa beim Autoklavieren wirkenden Temperaturbelastungen standhält.

Gemäß einer bevorzugten Ausführungsform der Erfindung ist das federnde Rastelement als Rasthülse ausgebildet, die einen oder mehrere radial außenseitig angeordnete, radial nach innen federnde Rasthaken aufweist. Die Rasthaken können federelastisch in die mindestens eine Rastausnehmung der Innenhülse des Endoskopkopfs einrasten. Vorzugsweise ist die Rasthülse sowohl innen- wie außenseitig im Wesentlichen zylindrisch ausgebildet und am proximalseitigen Rand ringförmig verdickt sowie am distalseitigen Rand mit den außenseitig vorstehenden Rasthaken ausgebildet. Das gemäß dieser Ausführungsform hülsenförmige Rastelement kann beispielsweise proximalseitig ringförmig geschlossen sein, jedoch distalseitig aus distaler Richtung eingreifende Schlitze aufweisen, die den distalen Abschnitt der Rasthülse in eine Mehrzahl von federnden Rastsegmenten unterteilen, die jeweils an ihrem distalen Rand die außenseitig vorstehenden Rasthaken aufweisen. Die distale Seite der Rasthülse kann als Widerlager für die Druckfeder dienen, die das Systemrohr in distaler Richtung belastet. Ein derartiges Rastelement ist einfach herstellbar und erlaubt eine sichere und leicht auszuführende Fixierung des Systemrohrs in axialer Richtung. Weiterhin kann eine solche Rasthülse ein Lösen der Fixierung zum Entnehmen des Systemrohrs aus dem Endoskopgehäuse ermöglichen, indem beispielsweise die Rastsegmente der Rasthülse zusammengedrückt werden, bis die Rasthaken aus den Rastausnehmungen bzw. aus der Rastnut ausgerückt sind und die Rasthülse abgezogen werden kann.

Gemäß einer weiteren bevorzugten Ausführungsform der Erfindung ist das federnde Rastelement als federnder Spannring ausgebildet. Der federnde Spannring kann beispielsweise als unterbrochener Ring oder auch nach Art eines Sprengrings ausgebildet sein und kann in seinem unterbrochenen Bereich elastisch zusammendrückbar sein, um den Außendurchmesser des Spannrings zu verringern; dies erleichtert das Einführen des Spannrings in das Endoskopgehäuse. Zum Fixieren des Systemrohrs rastet der Spannring durch elastische Rückverformung in die mindestens eine Rastausnehmung ein, beispielsweise in die innenseitige Rastnut der Innenhülse des Endoskopkopfs. Hierdurch wird eine besonders einfache Ausführungsform geschaffen, die ebenfalls eine sichere und leicht auszuführende Fixierung des Systemrohrs in axialer Richtung ermöglicht. Ferner kann hierdurch ein Lösen der Fixierung zum Entnehmen des Systemrohrs aus dem Endoskopgehäuse ermöglicht werden, indem beispielsweise der Spannring zusammengedrückt wird, bis er aus der Rastnut ausrückt und in proximaler Richtung abgezogen werden kann. Hierfür können bei einem als unterbrochener Ring ausgebildeten Spannring an den beiden Enden des Rings Ösen angeordnet sein, die mit einem entsprechenden Werkzeug gegriffen und zusammengezogen werden können.

In vorteilhafter Weise kann es vorgesehen sein, dass das federnde Rastelement eine oder mehrere Rastnasen zur Verdrehsicherung des Systemrohrs gegen eine axiale Rotation gegenüber dem Endoskopgehäuse aufweist. Diese können innen- und/oder außenseitig angeordnet sein und sind nicht umlaufend ausgebildet. Das Systemrohr bzw. das mit diesem verbundene Kopplungselement kann entsprechende Ausnehmungen aufweisen, in die die innenseitigen Rastnasen eingreifen; ebenso kann das Endoskopgehäuse bzw. die mit diesem verbundene Innenhülse Ausnehmungen aufweisen, in die außenseitig angeordnete Rastnasen eingreifen, welche zugleich als Rasthaken zur axialen Fixierung dienen können. Hierdurch kann eine verbesserte Justage zur Erzielung einer verbesserten Abbildungsqualität des Endoskops ermöglicht werden.

Vorzugsweise weist das federnde Rastelement mindestens ein umlaufendes Dichtelement auf, etwa einen O-Ring oder eine Dichtlippe. Dieser bzw. diese kann beispielsweise an einem ringförmigen Abschnitt eines als Rasthülse ausgebildeten Rastelements anliegen, so dass dann, wenn das Rastelement sich in einer Rastposition zum Herstellen der Rastverbindung befindet, das Dichtelement den Zwischenraum, in den das Rastelement eingesetzt ist, dicht abschließt. Auf diese Weise wird es ermöglicht, dass durch das Rastelement zugleich ein Innenraum des Endoskops gegen Eindringen von Feuchtigkeit abgedichtet wird. Eine solche Abdichtung kann zusätzlich zu einer geschlossenen Ausführung des Endoskopkopfs vorteilhaft sein, um ein Eindringen von Feuchtigkeit in das optische System zu vermeiden.

In vorteilhafter Weise kann das Rastelement eine oder mehrere Ausnehmungen zur Aufnahme von Trockenelementen aus einem geeigneten Trockenmittel aufweisen. Wenn beim Einsetzen des Rastelements dieses mit dem Trockenmittel bzw. den Trockenelementen befüllt ist, so kann auf einfache Weise ein verbesserter Schutz gegen ein Beschlagen optischer Bauteile durch etwaige eingedrungene Feuchtigkeit vermieden werden. Dies gilt in besonderem Maße, wenn das Rastelement zusätzlich ein umlaufendes Dichtelement aufweist.

Gemäß einem erfindungsgemäßen Verfahren zum Herstellen eines Endoskops, das einen langerstreckten Schaft und einen an einem proximalen Ende des Schafts angeordneten Endoskopkopf umfasst, wird ein Endoskopgehäuse bereitgestellt, das ein Außenrohr des Schafts und den am proximalen Ende des Schafts mit diesem verbundenen Endoskopkopf umfasst. Der Endoskopkopf umfasst eine Innenhülse, die auf Ihrer Innenseite mindestens eine Rastausnehmung aufweist, die beispielsweise als zumindest abschnittsweise umlaufende Nut ausgebildet sein kann. Der Endoskopkopf kann ein Gehäuse aufweisen, innerhalb dessen die Innenhülse angeordnet ist. Das Außenrohr, das Gehäuse des Endoskopkopfs und die Innenhülse sind vorzugsweise beim Bereitstellen des Endoskopgehäuses als Baugruppe vormontiert und fest miteinander verbunden. Das bereitgestellte Endoskopgehäuse kann weitere, ebenfalls bereits vormontierte, Bauelemente umfassen, beispielsweise einen Anschlussstutzen zum Anschließen eines Lichtleitkabels, mit dem Beleuchtungslicht von einer separaten Lichtquelle zugeführt werden kann, sowie Beleuchtungslichtleiter zum Weiterleiten des Beleuchtungslichts zum distalen Ende des Schafts.

Weiterhin wird als vormontierte Baugruppe eine Systemvorstufe bereitgestellt, die ein Systemrohr mit einem in diesem aufgenommenen Bildweiterleiter sowie ein fest mit dem proximalen Ende des Systemrohrs verbundenes Kopplungselement umfasst. Der Bildweiterleiter kann beispielsweise als Stablinsenanordnung ausgebildet sein, wobei die Stablinsen mit zwischen diesen angeordneten Abstandshaltern hintereinander im Systemrohr angeordnet sind. Vorzugsweise sind im distalen Endbereich des Systemrohrs oder in einer am distalen Ende des Systemrohrs mit diesem verbundenen Objektivhülse ein oder mehrere Linsenelemente eines Objektivs aufgenommen, an die proximalseitig der Bildweiterleiter bzw. die Stablinsenanordnung anschließt. Das Kopplungselement ist vorzugsweise im Wesentlichen hülsenförmig ausgebildet und außenseitig auf den proximalen Endbereich des Systemrohrs aufgesetzt. Die Systemvorstufe kann weitere, mit dem Systemrohr verbundene vormontierte Bauteile umfassen.

Erfindungsgemäß wird aus proximaler Richtung auf das Kopplungselement eine Druckfeder, beispielsweise eine Schraubenfeder, aufgeschoben, wobei der Verschiebeweg der Druckfeder durch ein Widerlager des Kopplungselements begrenzt ist, das beispielsweise als außenseitiger Ringbund ausgebildet sein kann. Weiter wird aus proximaler Richtung ein federndes Rastelement auf das Kopplungselement aufgeschoben, so dass die Druckfeder zwischen dem Widerlager des Kopplungselements und dem Rastelement angeordnet ist. Weiter wird eine Okularfassung am proximalen Ende des Kopplungselements angeschraubt. Die Okularfassung kann beispielsweise im Wesentlichen hülsenförmig ausgebildet sein und in ihrem distalen Endbereich ein Außengewinde tragen, mit dem die Okularfassung in ein entsprechendes Innengewinde des Kopplungselements eingeschraubt wird. Die Okularfassung kann derart ausgebildet sein, dass durch Anschrauben der Okularfassung zugleich das Rastelement und damit auch die von diesem gehaltene Druckfeder gegen Verlieren gesichert sind. Die auf diese Weise montierte Baugruppe, die die Systemvorstufe, die Druckfeder, das Rastelement und die Okularfassung umfasst, wird im Folgenden als Optikeinheit bezeichnet.

In einem weiteren Montageschritt wird die Optikeinheit und damit die Systemvorstufe in distaler Richtung in das Endoskopgehäuse bis zum Erreichen eines Anschlags eingeschoben. Nach dem Einschieben ist das Systemrohr innerhalb des Außenrohrs des Schafts angeordnet, reicht jedoch mit seinem proximalen Endbereich bis in den Endoskopkopf hinein. Das Kopplungselement, das mit dem proximalen Ende des Systemrohrs verbunden ist, sowie die auf dieses aufgeschobene Druckfeder und das Rastelement sind nun innerhalb des Gehäuses des Endoskopkopfs angeordnet, zumindest teilweise auch innerhalb der Innenhülse des Endoskopkopfs. Der in distaler Richtung die Einschubtiefe des Systemrohrs begrenzende Anschlag befindet sich vorzugsweise im distalen Endbereich des Schafts.

Weiter wird das federnde Rastelement in distaler Richtung bis zum Einrasten in die mindestens eine Rastausnehmung der Innenhülse vorgeschoben. Hierfür kann die Innenhülse in einem proximalen Abschnitt konisch ausgebildet sein, so dass das federnde Rastelement beim Vorschieben zusammengedrückt wird; durch Einschieben in den sich konisch verjüngenden Abschnitt der Innenhülse werden somit beispielsweise die Rastsegmente eines als Rasthülse ausgebildeten Rastelements radial nach innen zusammengedrückt bzw. die Enden eines als unterbrochener Ring ausgebildeten Spannrings werden zusammengedrückt. Bei Erreichen der mindestens einen Rastausnehmung bewirkt die elastische Rückverformung des Rastelements ein Einrasten in die Rastausnehmung, so dass nun beispielsweise die Rasthaken der Rasthülse bzw. der Spannring selbst in die Rastausnehmung eingreifen und die Optikeinheit in axialer Richtung halten. Die Optikeinheit wird dadurch gegen eine Verschiebung in proximaler Richtung durch das eingerastete Rastelement gehalten, während der erwähnte Anschlag eine Verschiebung in distaler Richtung begrenzt.

Durch das Zusammenwirken des eingerasteten Rastelements mit der Druckfeder und dem Anschlag wird somit die Systemvorstufe sowie die Optikeinheit insgesamt auf einfache Weise in ihrer axialen Position festgelegt. Durch die Auslegung der Druckfeder kann dabei die über das Systemrohr auf den Anschlag wirkende Kraft begrenzt werden. Durch das erfindungsgemäße Verfahren kann eine vereinfachte Montage der Optikeinheit im Endoskopgehäuse ermöglicht werden, beispielsweise ohne Verschrauben oder Verschweißen zum Fixieren der Systemvorstufe. Hierdurch wird es insbesondere ermöglicht, das Endoskopgehäuse und die Systemvorstufe bzw. die Optikeinheit getrennt zu fertigen und vorzumontieren und danach unter einem minimalen Justage- und Montageaufwand miteinander zu fügen.

Gemäß einer bevorzugten Ausführungsform der Erfindung umfasst das Endoskopgehäuse ein Innenrohr, das im Außenrohr des Schafts verläuft und das fest mit der Innenhülse des Endoskopkopf verbunden ist; in einem Zwischenraum zwischen dem Außenrohr und dem Innenrohr können beispielsweise Beleuchtungslichtleiter aufgenommen sein. Beim Einschieben der vormontierten Optikeinheit in das vormontierte Endoskopgehäuse wird somit das Systemrohr in das Innenrohr eingeschoben, bis das Systemrohr den Anschlag für das Systemrohr erreicht. Der Anschlag kann dabei in vorteilhafter Weise durch ein im distalen Endbereich des Innenrohrs befestigtes Deckglas gebildet werden. Das Systemrohr bzw. die Optikeinheit wird in diesem Fall so weit in das Innenrohr eingeschoben, bis das distale Ende des Systemrohrs oder ein mit diesem verbundenes Bauelement, beispielsweise eine Objektivhülse oder ein von dieser gehaltenes Linsenelement eines Objektivs, an dem Deckglas oder an einem Bauelement, das sich an dem Deckglas abstützt, anliegt. Durch das Einschieben des federnden Rastelements bis zum Einrasten in die mindestens eine Rastausnehmung wird das Systemrohr somit durch die Druckfeder gegen das Deckglas vorgespannt. Hierdurch kann eine besonders einfache und sichere Fixierung des Systemrohrs in axialer Richtung erreichbar sein.

Weiterhin ist es bevorzugt, dass ein Okular in die Okularfassung eingesetzt wird; dies kann vor oder nach dem Anschrauben der Okularfassung an das proximale Ende des Kopplungselements erfolgen. Das Okular kann aus einer Okularhülse und einer oder mehreren in dieser aufgenommenen Okularlinsen bestehen. Nach dem Anschrauben der Okularfassung und vor dem Einschieben der Optikeinheit in das Endoskopgehäuse wird das Okular relativ zum übrigen optischen System des Endoskops, insbesondere relativ zum Bildweiterleiter, justiert; hierfür kann die Okularfassung entsprechende Justiermittel, etwa Justierschrauben, umfassen. Die Optikeinheit, die im nachfolgenden Schritt in das Endoskopgehäuse eingeschoben wird, umfasst in diesem Fall auch das Okular, das bereits justiert ist. Hierdurch wird eine weitere Vereinfachung der Montage des Endoskops ermöglicht.

Zusätzlich zu den genannten Herstellungsschritten können weitere Herstellungsschritte vorgesehen sein. So kann insbesondere nach dem Fixieren der Optikeinheit im Endoskopgehäuse durch Einrasten des Rastelements der Endoskopkopf verschlossen werden, indem proximalseitig eine Okularmuschel und ein Okulardeckglas aufgesetzt und dicht mit dem Gehäuse des Endoskopkopfs verbunden werden. Vorzugsweise wird das Endoskop hermetisch dicht abgeschlossen.

Weiterhin kann gemäß der vorliegenden Erfindung eine besonders einfache Demontage des Endoskops ermöglicht werden. Hierfür kann es vorgesehen sein, dass das Gehäuse des Endoskopkopfs proximalseitig geöffnet werden kann, so dass die Okularfassung zugänglich wird. Zur Demontage des Endoskops kann sodann die Okularfassung aus dem Kopplungselement herausgeschraubt werden. Durch Zusammendrücken des Rastelements, beispielsweise der Rastsegmente einer Rasthülse oder der Enden eines als unterbrochener Ring ausgebildeten Spannrings, kann das Rastelement aus der mindestens einen Rastausnehmung der Innenhülse ausgerastet und in proximaler Richtung aus dem Zwischenraum zwischen der Innenhülse und dem Kopplungselement herausgezogen werden; hierfür kann ein entsprechend ausgebildetes Werkzeug erforderlich sein. Nach dem Entnehmen des Rastelements kann sodann die Systemvorstufe aus dem Endoskopgehäuse herausgezogen werden. Hierdurch wird auf besonders einfache Weise ein Austausch oder eine Reparatur der Systemvorstufe ermöglicht.

Ein Rastelement für ein Endoskop ist als federndes Rastelement zum Herstellen einer Rastverbindung zum Halten des Systemrohrs eines Endoskops in einer axialen Richtung des Schafts des Endoskops ausgebildet. Insbesondere ist das Rastelement wie zuvor beschrieben ausgebildet.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind.

Weitere Aspekte der Erfindung ergeben sich aus der nachfolgenden Beschreibung zweier bevorzugter Ausführungsbeispiele und der beigefügten Zeichnung. Es zeigen:
Fig. 1 ein erstes Ausführungsbeispiel eines erfindungsgemäßen Endoskops in einer teilweise aufgeschnittenen Seitenansicht;
Fig. 2 einen Teil des inneren Aufbaus des Endoskopkopfs des Endoskops gemäß Fig. 1 in einem Längsschnitt;
Fig. 3 einen Teil des inneren Aufbaus des Endoskopkopfs des Endoskops gemäß Fig. 1 in einer geschnittenen Seitenansicht;
Fig. 4 das distale Ende des Schafts des Endoskops gemäß Fig. 1 in einer teilweise aufgeschnittenen Seitenansicht;
Fig. 5 die Rasthülse des Endoskops gemäß Fig. 1 in einer Schrägansicht;
Fig. 6 ein zweites Ausführungsbeispiel eines erfindungsgemäßen Endoskops in einer der Fig. 1 entsprechenden Darstellung;
Fig. 7 einen Teil des inneren Aufbaus des Endoskopkopfs des Endoskops gemäß Fig. 6 in einem Längsschnitt;
Fig. 8 einen Teil des inneren Aufbaus des Endoskopkopfs des Endoskops gemäß Fig. 6 in einer Schrägansicht;
Fig. 9 den Spannring des Endoskops gemäß Fig. 6 in einer Schrägansicht.

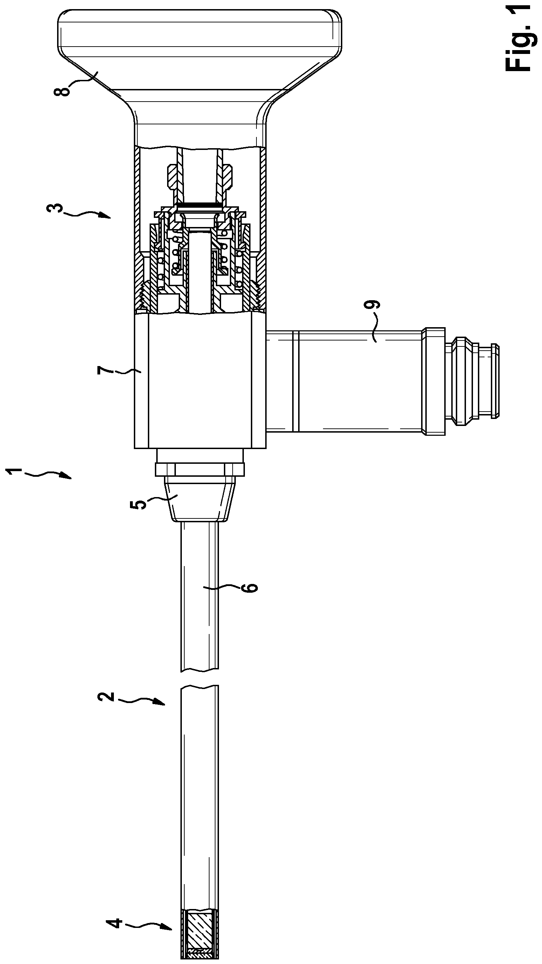

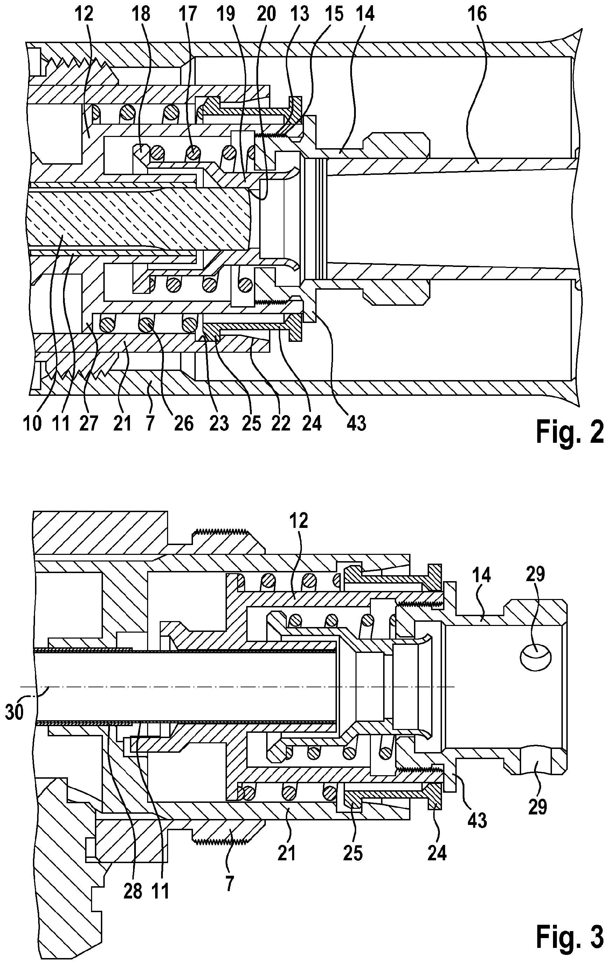

In Fig. 1 ist ein Endoskop gemäß einer ersten Ausführungsform der Erfindung in einer teilweise aufgeschnittenen Seitenansicht gezeigt. Das Endoskop 1 umfasst einen langerstreckten Schaft 2 und einen am proximalen Ende des Schafts 2 angeordneten Endoskopkopf 3. Der distale Endbereich 4 des Endoskopschafts 2 ist in Fig. 1 aufgeschnitten dargestellt. Der Endoskopkopf 3, der in Fig. 1 ebenfalls teilweise aufgeschnitten dargestellt ist, ist über einen Konus 5 mit einem Außenrohr 6 des Schafts verbunden. Der Endoskopkopf 3 weist ein Gehäuse 7 auf, an dessen proximalem Ende eine Okularmuschel 8 angesetzt ist. Weiter trägt das Gehäuse 7 des Endoskopkopfs 3 einen Lichtanschlussstutzen 9. In den in Fig. 1 aufgeschnitten dargestellten Abschnitten des Endoskops 1 sind weitere Bauelemente angedeutet, die in den Figuren 2 bis 5 vergrößert gezeigt und im Folgenden näher beschrieben sind.

In Fig. 2 ist ein Teil des inneren Aufbaus des Endoskopkopfs 3 des Endoskops 1 in einem Längsschnitt dargestellt. Bei den hier beschriebenen Ausführungsbeispielen der Erfindung ist der Bildweiterleiter als System von hintereinander angeordneten Stablinsen ausgebildet, wovon der proximale Abschnitt der proximalen Stablinse 10 in Fig. 2 zu erkennen ist. Die in ihrem mittleren Abschnitt verjüngte Stablinse 10 ist in einem Systemrohr 11 aufgenommen, das auch die weiteren, in distaler Richtung vorgeschalteten und in Fig. 2 nicht dargestellten Stablinsen aufnimmt. Das Systemrohr 11 ist durch Verschweißen mit einem auf das proximale Ende des Systemrohrs 11 aufgesetzten, näherungsweise hülsenförmigen Kopplungselement 12 verbunden. In seinem proximalen Endbereich weist der äußere hülsenförmige Teil des Kopplungselements 12 ein Innengewinde 13 auf, in das eine näherungsweise hülsenförmige Okularfassung 14 mit einem Außengewinde 15 eingeschraubt ist. In der Okularfassung 14 ist eine Okularhülse 16, die in Fig. 2 nicht dargestellte optische Elemente des Okulars umfasst, justierbar gehalten. Distalseitig liegt an der Okularfassung 14 eine als Schraubendruckfeder ausgebildete Optikfeder 17 an, die sich distalseitig an einem Ringbund 18 einer Stablinsenfassung 19 abstützt. In der Stablinsenfassung 19 ist das proximale Ende der proximalen Stablinse 10 des Bildweiterleiters gehalten. Über einen in Fig. 2 nicht dargestellten Abstandshalter, der an einem Absatz 20 der Stablinsenfassung anliegt, wird die Stablinse 10 von der Optikfeder 17 in distaler Richtung belastet; die distale Seite der Okularfassung 14 dient dabei für die Optikfeder 17 als Widerlager. Da die weiteren distalseitig auf die Stablinse 10 folgenden Stablinsen über Abstandshalter an der Stablinse 10 und aneinander anliegen, wird das gesamte Stablinsensystem hierdurch in distaler Richtung gegen einen distalen Anschlag des Stablinsensystems vorgespannt und dadurch in axialer Richtung fixiert. In radialer Richtung, d.h. in Querrichtung des Endoskops 1, können die Stablinse 10 sowie die weiteren Stablinsen und Bauelemente durch enge Toleranzen fixiert sein.

Wie in Fig. 2 weiter gezeigt ist, ist in dem Gehäuse 7 des Endoskopkopfs 3 eine Innenhülse 21 angeordnet, die fest mit dem Gehäuse 7 verbunden ist. Die Innenhülse 21 weist in ihrem proximalen Endabschnitt innenseitig eine sich nach distal verengende konische Verjüngung 22 auf, distalseitig von welcher eine umlaufende Rastnut 23 angeordnet ist, die als innenseitiger Einstich der Innenhülse 21 ausgebildet ist. Außenseitig auf das Kopplungselement 12 ist ein Rastelement aufgeschoben, das bei dem dargestellten Ausführungsbeispiel als Rasthülse 24 ausgebildet ist. Die Rasthülse 24 ist in ihrem proximalen Endbereich ringförmig geschlossen und weist in ihrem distalen Endbereich federnd gehaltene Rasthaken 25 auf, die in die Rastnut 23 eingreifen. Wie in Fig. 2 weiter dargestellt ist, bildet die distale Seite der Rasthülse 24 ein Widerlager für eine als Schraubenfeder ausgebildete Druckfeder 26, die sich distalseitig an einem Ringbund 27 des Kopplungselements 12 abstützt. Da die Rasthülse 24 in der Nut 23 gegen ein Verschieben in proximaler Richtung gehalten ist, wird das Kopplungselement 12 und somit das Systemrohr 11 durch die Druckfeder 26 in distaler Richtung gegen einen weiter unten beschriebenen Anschlag für das Systemrohr 11 gedrückt und dadurch in axialer Richtung fixiert. Durch die Auslegung der Optikfeder 17 und der Druckfeder 26 können die zur Fixierung der Bauelemente auf das Stablinsensystem bzw. das Systemrohr 11 jeweils wirkenden Kräfte eingestellt werden.

In Fig. 3 ist der innere Aufbau des Endoskopkopfs 3 in einer geschnittenen Seitenansicht dargestellt, wobei das aus mehreren Bauteilen bestehende Gehäuse 7 des Endoskopkopfs 3 nur teilweise dargestellt ist. Wie in Fig. 3 gezeigt ist, ist die Innenhülse 21 fest mit dem Gehäuse 7 verbunden. Die Innenhülse 21 ist wiederum fest mit einem Innenrohr 28 verbunden, beispielsweise verlötet, in dem das Systemrohr 11 geführt ist. Ferner sind in Fig. 3 zwei von drei Justierbohrungen 29 zu erkennen, in die Justierschrauben zur Justierung der Okularhülse 16 relativ zur Stablinse 10 bzw. relativ zur optischen Achse 30 eingesetzt werden können. Die Stablinse 10 und die Okularhülse 16 sind in Fig. 3 nicht gezeigt.

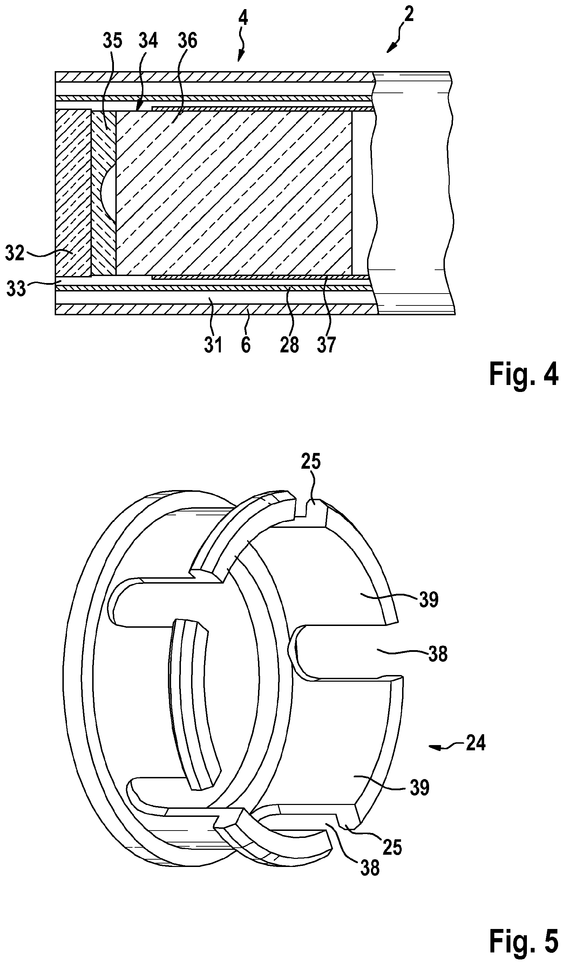

In Fig. 4 ist der distale Endbereich 4 des Schafts 2 des Endoskops 1 gemäß Fig. 1 aufgeschnitten und vergrößert dargestellt. Wie in Fig. 4 zu erkennen ist, verläuft innerhalb des Außenrohrs 6 das Innenrohr 28. In dem Zwischenraum 31 zwischen dem Außenrohr 6 und dem Innenrohr 28 sind nicht dargestellte Beleuchtungslichtleiter angeordnet. Als distalseitiger Abschluss des vom Innenrohr 28 umschlossenen Lumens ist ein Deckglas 32 in das Innenrohr 28 eingelötet, wobei der Zwischenraum 33, der mit Lot gefüllt ist, in Fig. 4 der Deutlichkeit halber verbreitert dargestellt ist. In proximaler Richtung folgen auf das Deckglas 32 das Objektiv 34 und die Stablinsen des in Fig. 4 nicht gezeigten Bildweiterleiters. Das Objektiv 34 umfasst eine erste Objektivlinse 35 sowie eine Objektivlinseneinheit 36, die weitere in Fig. 4 nicht im Einzelnen dargestellte Linsenelemente umfasst. Das Deckglas 32, die Objektivlinse 35 und die weiteren genannten Linsenelemente können durch in Fig. 4 nicht dargestellte Abstandshalter voneinander getrennt sein. Die Objektivlinseneinheit 36 ist durch Verkleben fest in eine Objektivhülse 37 eingesetzt, die fest mit dem distalen Ende des Systemrohrs 11, das nicht notwendig bis in den in Fig. 4 gezeigten Endbereich 4 hineinreicht, verbunden ist.

Wie zu Fig. 2 beschrieben, wird das Stablinsensystem durch die Optikfeder 17 gegen einen distalen Anschlag des Stablinsensystems belastet. Dieser Anschlag, gegen den das Stablinsensystem axial innerhalb des Systemrohrs 11 fixiert wird, wird durch die fest mit dem distalen Ende des Systemrohrs 11 verbundene Objektivlinseneinheit 36 gebildet. Das Systemrohr 11 wird durch die Druckfeder 26 in distaler Richtung gegen einen Anschlag für das Systemrohr 11 belastet, der durch das fest im Innenrohr 28 gehaltene Deckglas 32 bzw. die an diesem ggf. über Abstandshalter anliegende erste Objektivlinse 35 gegeben ist. Dieser Anschlag ist für das Systemrohr 11 erreicht, wenn die Objektivlinseneinheit 36, ggf. über einen Abstandshalter, an der ersten Objektivlinse 35 anliegt. Hierdurch wird das Systemrohr 11 sowie das in diesem aufgenommene optische System in axialer Richtung innerhalb des Endoskops 1 fixiert.

In Fig. 5 ist die Rasthülse 24 in einer vergrößerten, schräg aus distaler Richtung gesehenen Ansicht gezeigt. Wie in Fig. 5 zu erkennen ist, ist das distale Ende der Rasthülse 24 durch Schlitze 38 in eine Mehrzahl von federnden Rastsegmenten 39 geteilt. Die Rastsegmente 39 tragen an ihrem distalen Ende die nach außen gerichteten Rasthaken 25. Proximalseitig ist die Rasthülse 24 als geschlossener Ring ausgebildet.

Bei der Herstellung des wie zuvor beschrieben ausgebildeten Endoskops 1 werden das Endoskopgehäuse, das das Außenrohr 6, das Gehäuse 7 des Endoskopkopfs 3, die Innenhülse 21 und das Innenrohr 28 umfasst, vormontiert und bereitgestellt. Das vormontierte Endoskopgehäuse umfasst ebenfalls den Lichtanschlussstutzen 9 und die durch diesen bis zum distalen Endbereich 4 des Endoskops 1 geführten Beleuchtungslichtleiter sowie ggf. weitere Bauteile. Weiterhin ist bei dem vormontierten Endoskopgehäuse das Deckglas 32 in das Innenrohr 28 an dessen distalem Ende eingelötet. Ferner wird, ebenfalls als vormontierte Baugruppe, die Systemvorstufe bereitgestellt, die das Systemrohr 11, die in diesem aufgenommenen Stablinsen und Abstandshalter, das mit dem Systemrohr 11 verschweißte Kopplungselement 12 und ggf. weitere Bauteile umfasst. Die Systemvorstufe umfasst auch die Objektivlinseneinheit 36, die in die Objektivhülse 37 eingeklebt ist, wobei die Objektivhülse 37 fest mit dem distalen Ende des Systemrohrs 11 verbunden ist. Weiter wird eine Okularbaugruppe bereitgestellt, die die Okularfassung 14, die in diese eingesetzte und proximalseitig umgebördelte Stablinsenfassung 19 und die auf der Außenseite der Stablinsenfassung 19 angeordnete Optikfeder 17 umfasst.

Zur Montage des Endoskops 1 werden aus proximaler Richtung auf das Kopplungselement 12 zunächst die Druckfeder 26 und danach die Rasthülse 24 aufgeschoben. Die Okularfassung 14 wird nun mit ihrem Außengewinde 15 in das Innengewinde 13 des Kopplungselements eingeschraubt, wobei das Kopplungselement 12 gegen die Scheibe 43 der Okularfassung 14 angezogen wird. Hierbei gelangt die proximale Stablinse 10 unter die von der Optikfeder 17 über die Stablinsenfassung 19 ausgeübte Vorspannungskraft, wodurch das Stablinsensystem gegen die Objektivlinseneinheit 36 gedrückt und dadurch fixiert wird. Die Rasthülse 24 liegt nach dem Einschrauben der Okularhülse 14 in das Kopplungselement 12 an der Scheibe 43 der Okularfassung 14 an und ist durch diese auch gegen Verlieren gesichert. Die Okularhülse 16 mit den in dieser aufgenommenen Okularlinsen wird in die Okularfassung 14 eingesetzt und mittels durch die Justierbohrungen 29 geführten Justierschrauben relativ zur optischen Achse 30 des Stablinsensystems justiert. Der Zusammenbau aus der Systemvorstufe mit der Druckfeder 26 und der Rasthülse 24 sowie der Okularbaugruppe stellt die Optikeinheit des Endoskops 1 dar, die nahezu das gesamte optische System des Endoskops 1 umfasst.

Die Optikeinheit wird nun aus proximaler Richtung in das vormontierte Endoskopgehäuse eingeschoben, wobei das Systemrohr 11 in das Innenrohr 28 eingeschoben wird. Dabei werden die Rasthaken 25 über die Verjüngung 22 der Innenhülse 21 nach innen gedrückt, rasten aber noch nicht in die Rastnut 23 ein. Nachdem die mit dem Innenrohr 21 verbundene Objektivlinseneinheit 26 den durch das Deckglas 32 und die erste Objektivlinse 35 gebildeten Anschlag erreicht hat, wird die Rasthülse 24 weiter in proximaler Richtung verschoben, bis die Rasthaken 25 in die Rastnut 23 einrasten. Hierbei wird die Druckfeder 26 komprimiert, die dadurch die zur axialen Fixierung der Optikeinheit erforderliche Kraft erzeugt. Die Optikeinheit ist auf diese Weise gegen axiales Verschieben gesichert. Da das Systemrohr 11 mit engen Toleranzen innerhalb des Innenrohrs 28 geführt ist, ist die Optikeinheit insgesamt innerhalb des Endoskops 1 ausreichend festgelegt; ein Einstellen oder ein zusätzliches Arretieren entfällt. Zusätzlich kann ein Fixieren der Optikeinheit gegen eine Rotation um die Längsachse des Endoskops 1 erfolgen; dies kann aber auch entbehrlich sein. Ferner können weitere Arbeitsschritte, wie etwas das Einbringen von Trockenmittel, erfolgen. Schließlich wird der Endoskopkopf 3 durch Aufsetzen der Okularmuschel 8 und des Okulardeckglases dicht abgeschlossen.

Zum Entnehmen der Systemvorstufe kann der Endoskopkopf 3 geöffnet und die Okularfassung 14 aus dem Kopplungselement 12 herausgeschraubt werden. Die Rastsegmente 39 der Rasthülse 24 werden über ein in die Verjüngung 22 eingeschobenes Werkzeug zusammengedrückt, bis die Rasthaken 25 aus der Rastnut 23 ausrücken. Sodann kann die Rasthülse 24 in proximaler Richtung abgezogen und die Systemvorstufe aus dem Endoskopgehäuse herausgezogen werden.

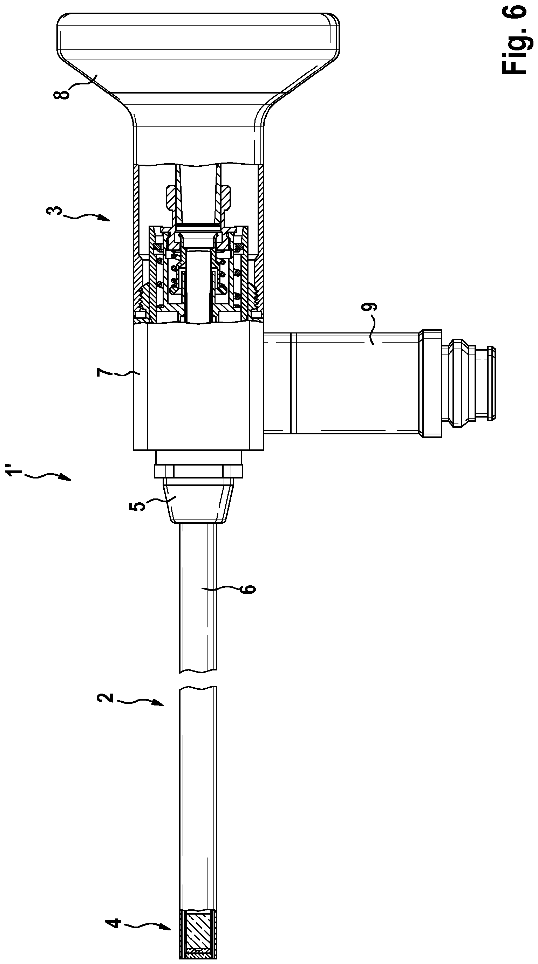

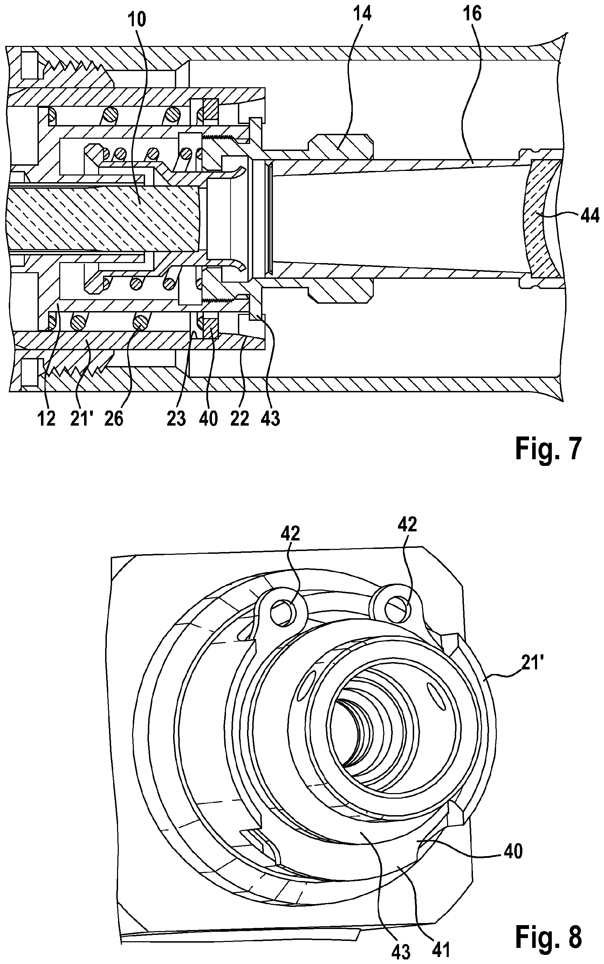

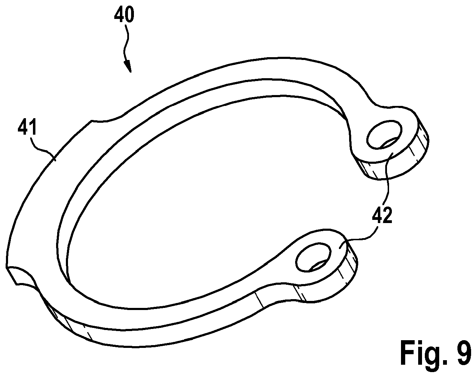

In Fig. 6 ist in einer der Fig. 1 entsprechenden Ansicht ein Endoskop 1' gemäß einem zweiten Ausführungsbeispiel der Erfindung gezeigt. Wie in dem in Fig. 7 dargestellten Längsschnitt durch den Endoskopkopf 3 gezeigt ist, ist hierbei anstelle der Rasthülse 24 ein elastisch verformbarer, als unterbrochener Ring ausgebildeter Spannring 40 vorgesehen, der in die Rastnut 23 eingerastet ist und in entsprechender Weise wie zuvor beschrieben als proximales Widerlager der Druckfeder 26 dient und eine Fixierung der Optikeinheit im Endoskopgehäuse bewirkt. In Fig. 7 ist ferner exemplarisch eine in die Okularhülse 16 eingesetzte Okularlinse 44 gezeigt. Wie in Fig. 8 in einer aus proximaler Richtung gesehenen Schrägansicht zu erkennen ist, ist die Rastnut 23 bei dieser Ausführungsform nicht umlaufend ausgebildet, sondern die Innenhülse 21' weist umfangsmäßig zwei Unterbrechungen auf, durch die eine Verbreiterung 41 sowie zwei Ösen 42 des Spannrings 40, die auf beiden Seiten der Unterbrechung des Spannrings 40 angeordnet sind, hindurchragen. Der Spannring 40 mit der Verbreiterung 41 und den Ösen 42 ist in Fig. 9 in einer Schrägansicht gezeigt. Im Übrigen ist das Endoskop 1' gemäß dem zweiten Ausführungsbeispiel der Erfindung wie das zuvor beschriebene Endoskop 1 aufgebaut.

Zur Herstellung des Endoskops 1' wird wie zuvor zum ersten Ausführungsbeispiel der Erfindung beschrieben vorgegangen und in entsprechender Weise das Endoskopgehäuse, die Systemvorstufe und die Okulareinheit bereitgestellt. Auf das Kopplungselement 12 werden sodann die Druckfeder 26 und der Spannring 40 aufgeschoben. Nun wird die Okularfassung 14 in das Kopplungselement 12 eingeschraubt, wonach die Okularhülse 16 eingesetzt und das Okular justiert werden kann. Die derart vormontierte Optikeinheit kann nun in das Endoskopgehäuse eingeschoben werden, bis die Objektivlinseneinheit 36, wie zuvor beschrieben, den durch das Deckglas 32 bestimmten Anschlag erreicht. Nun wird der Spannring 40, der noch nicht in die Rastnut 23 eingerastet ist, sondern an der Scheibe 43 der Okularfassung 14 anliegt, zum Einrasten in distaler Richtung verschoben. Dabei wird der Spannring 40 über die Verjüngung 22 zusammengedrückt und rastet schließlich durch elastische Rückverformung in die Rastnut 32 ein. In entsprechender Weise wie zum ersten Ausführungsbeispiel für die Rasthülse 24 beschrieben, bildet der Spannring 40 das distalseitige Widerlager für die Druckfeder 26, die auf das Kopplungselement 12 und damit auf das Systemrohr 11 die zur axialen Festlegung erforderliche Kraft ausübt. In Querrichtung wird das Systemrohr 11 durch enge Toleranzen innerhalb des Innenrohrs 28 gehalten.

Zur Demontage kann die Okularfassung 14 aus dem Kopplungselement 12 herausgeschraubt werden. Sodann kann der Spannring 40 durch Zusammendrücken der Ösen 42 mittels eines Werkzeugs zusammengezogen, zum Ausrücken aus der Rastnut 32 gebracht und in proximaler Richtung abgezogen werden. Danach kann die Systemvorstufe entnommen werden, indem das Systemrohr 11 aus dem Innenschaft 28 herausgezogen wird.

Der Übersichtlichkeit halber sind nicht in allen Figuren alle Bezugszeichen dargestellt. Zu einer Figur nicht erläuterte Bezugszeichen haben die gleiche Bedeutung wie in den übrigen Figuren.

### Bezugszeichenliste

- 1, 1': Endoskop
- 2: Schaft
- 3: Endoskopkopf
- 4: Distaler Endbereich
- 5: Konus
- 6: Außenrohr
- 7: Gehäuse
- 8: Okularmuschel
- 9: Lichtanschlussstutzen
- 10: Stablinse
- 11: Systemrohr
- 12: Kopplungselement
- 13: Innengewinde
- 14: Okularfassung
- 15: Außengewinde
- 16: Okularhülse
- 17: Optikfeder
- 18: Ringbund
- 19: Stablinsenfassung
- 20: Absatz
- 21, 21': Innenhülse
- 22: Verjüngung
- 23: Rastnut
- 24: Rasthülse
- 25: Rasthaken
- 26: Druckfeder
- 27: Ringbund
- 28: Innenrohr
- 29: Justierbohrung
- 30: Optische Achse
- 31: Zwischenraum
- 32: Deckglas
- 33: Zwischenraum
- 34: Objektiv
- 35: Objektivlinse
- 36: Objektivlinseneinheit
- 37: Objektivhülse
- 38: Schlitz
- 39: Rastsegment
- 40: Spannring
- 41: Verbreiterung
- 42: Öse
- 43: Scheibe
- 44: Okularlinse

## Patentansprüche

1. Endoskop mit einem langerstreckten Schaft (2), einem Federelement, einem federelastisch ausgebildeten federnden Rastelement mit einem Widerlager für das Federelement und mit einem innerhalb des Schafts (2) verlaufenden Systemrohr (11), in dem ein Bildweiterleiter angeordnet ist, wobei das Systemrohr (11) mittels mindestens besagten federnden Rastelements in einer axialen Richtung des Schafts (2) gehalten ist, **dadurch gekennzeichnet, dass** das federnde Rastelement, Widerlager und das Federelement derart konfiguriert sind, dass das Systemrohr (11) durch das Federelement in einer distalen Richtung gegen einen Anschlag belastet wird.

2. Endoskop nach Anspruch 1, **dadurch gekennzeichnet, dass** der Anschlag im distalen Endbereich (4) des Schafts (2) ausgebildet ist.

3. Endoskop nach Anspruch 2, **dadurch gekennzeichnet, dass** der Anschlag durch ein im distalen Endbereich des Schafts (2) befestigtes Deckglas (32) gebildet wird, gegen das das Systemrohr (11) über ein zwischen dem Deckglas (32) und dem Bildweiterleiter angeordnetes Objektiv (34) anliegt.

4. Endoskop nach einem der vorhergehenden Ansprüche, **dadurch gekennzeichnet, dass** das Endoskop (1, 1') einen an einem proximalen Ende des Schafts (2) angeordneten Endoskopkopf (3) aufweist, dass sich das Systemrohr (11) bis in den Endoskopkopf (3) erstreckt und dass das federnde Rastelement im Endoskopkopf (3) aufgenommen ist und einen proximalen Endbereich des Systemrohrs (11) und ein Gehäuse (7) des Endoskopkopfs (3) verbindet.

5. Endoskop nach Anspruch 4, **dadurch gekennzeichnet, dass** das federnde Rastelement in mindestens eine auf einer Innenseite einer Innenhülse (21) des Endoskopkopfs (3) ausgebildete Rastausnehmung eingerastet ist.

6. Endoskop nach einem der vorhergehenden Ansprüche, **dadurch gekennzeichnet, dass** das Systemrohr (11) lösbar gehalten ist.

7. Endoskop nach einem der vorhergehenden Ansprüche, **dadurch gekennzeichnet, dass** das federnde Rastelement als Rasthülse (24) mit mindestens einem außenseitig angeordneten federnden Rasthaken (25) ausgebildet ist.

8. Endoskop nach einem der vorhergehenden Ansprüche, **dadurch gekennzeichnet, dass** das Rastelement als federnder Spannring (40) ausgebildet ist.

9. Endoskop nach einem der vorhergehenden Ansprüche, **dadurch gekennzeichnet, dass** das Rastelement eine oder mehrere Rastnasen zur Verdrehsicherung aufweist.

10. Endoskop nach einem der vorhergehenden Ansprüche, **dadurch gekennzeichnet, dass** das federnde Rastelement mindestens ein umlaufendes Dichtelement und/oder mindestens eine Ausnehmung zur Aufnahme von Trockenmittel aufweist.

11. Verfahren zum Herstellen eines Endoskops (1, 1') mit einem langerstreckten Schaft (2) und einem Endoskopkopf (3), umfassend folgende Schritte:
- Bereitstellen eines Endoskopgehäuses, das ein Außenrohr (6) des Schafts (2) und den an einem proximalen Ende des Schafts (2) mit diesem verbundenen Endoskopkopf (3) umfasst, welcher eine Innenhülse (21) aufweist, die auf einer Innenseite mindestens eine Rastausnehmung aufweist,
- Bereitstellen einer Systemvorstufe, die ein Systemrohr (11) mit einem in diesem aufgenommenen Bildweiterleiter und ein fest mit einem proximalen Ende des Systemrohrs (11) verbundenes Kopplungselement (12) umfasst,
- Aufschieben einer Druckfeder (26) und eines federnden Rastelements aus einer proximalen Richtung auf das Kopplungselement (12),
- Anschrauben einer Okularfassung (14) an einem proximalen Ende des Kopplungselements (12),
- Einschieben der Systemvorstufe in das Endoskopgehäuse in einer distalen Richtung bis zum Erreichen eines Anschlags und
- Verschieben des Rastelements in der distalen Richtung auf dem Kopplungselement (12) bis zum Einrasten des Rastelements in die mindestens eine Rastausnehmung.

12. Verfahren nach Anspruch 11, wobei das Endoskopgehäuse ein im Außenrohr (6) angeordnetes Innenrohr (28) umfasst, das fest mit der Innenhülse (21) des Endoskopkopfs (3) verbunden ist, wobei beim Einschieben der Systemvorstufe in das Endoskopgehäuse das Systemrohr (11) in das Innenrohr (28) eingeschoben wird und der Anschlag im distalen Endbereich des Innenrohrs (28) ausgebildet ist.

13. Verfahren nach Anspruch 11 oder 12, wobei ein Okular in die Okularfassung (14) eingesetzt und nach dem Anschrauben der Okularfassung (14) und vor dem Einschieben der Systemvorstufe in das Endoskopgehäuse justiert wird.

## Claims

1. Endoscope with an elongate shaft (2), a spring element, an elastically resilient latching element with an abutment for the spring element, and with a system tube (11) which extends inside the shaft (2) and in which an image carrier is arranged, wherein the system tube (11) is held in an axial direction of the shaft (2) by means of at least said resilient latching element, **characterized in that** the resilient latching element, the abutment and the spring element are configured in such a way that the system tube (11) is loaded by the spring element in a distal direction against a limit stop.

2. Endoscope according to Claim 1, **characterized in that** the limit stop is formed in the distal end region (4) of the shaft (2).

3. Endoscope according to Claim 2, **characterized in that** the limit stop is formed by a cover glass (32) which is secured in the distal end region of the shaft (2) and against which the system tube (11) bears via an objective (34) arranged between the cover glass (32) and the image carrier.

4. Endoscope according to one of the preceding claims, **characterized in that** the endoscope (1, 1') has an endoscope head (3) arranged at a proximal end of the shaft (2), **in that** the system tube (11) extends into the endoscope head (3), and **in that** the resilient latching element is received in the endoscope head (3) and connects a proximal end region of the system tube (11) and a housing (7) of the endoscope head (3) .

5. Endoscope according to Claim 4, **characterized in that** the resilient latching element is latched into at least one latching recess formed on an inner face of an inner sleeve (21) of the endoscope head (3).

6. Endoscope according to one of the preceding claims, **characterized in that** the system tube (11) is held releasably.

7. Endoscope according to one of the preceding claims, **characterized in that** the resilient latching element is designed as a latching sleeve (24) with at least one resilient latching hook (25) arranged on the outside.

8. Endoscope according to one of the preceding claims, **characterized in that** the latching element is designed as a resilient clamping ring (40).

9. Endoscope according to one of the preceding claims, **characterized in that** the latching element has one or more latching lugs for securing against rotation.

10. Endoscope according to one of the preceding claims, **characterized in that** the resilient latching element has at least one peripheral sealing element and/or at least one recess for receiving desiccant.

11. Method for producing an endoscope (1, 1') with an elongate shaft (2) and an endoscope head (3), said method comprising the following steps:
- making available an endoscope housing which comprises an outer tube (6) of the shaft (2) and the endoscope head (3) connected thereto at a proximal end of the shaft (2), which endoscope head (3) has an inner sleeve (21), which has at least one latching recess on an inner face,
- making available a system precursor which comprises a system tube (11) with an image carrier received therein, and a coupling element (12) fixedly connected to a proximal end of the system tube (11),
- pushing a compression spring (26) and a resilient latching element onto the coupling element (12) from a proximal direction,
- screwing an eyepiece mount (14) onto a proximal end of the coupling element (12),
- pushing the system precursor into the endoscope housing in a distal direction until a limit stop is reached, and
- moving the latching element in the distal direction on the coupling element (12) until the latching element latches into the at least one latching recess.

12. Method according to Claim 11, wherein the endoscope housing comprises an inner tube (28) which is arranged in the outer tube (6) and which is fixedly connected to the inner sleeve (21) of the endoscope head (3), wherein, upon insertion of the system precursor into the endoscope housing, the system tube (11) is pushed into the inner tube (28) and the limit stop is formed in the distal end region of the inner tube (28).

13. Method according to Claim 11 or 12, wherein an eyepiece is inserted into the eyepiece mount (14) and is adjusted after the eyepiece mount (14) has been screwed on and before the system precursor is pushed into the endoscope housing.

## Revendications

1. Endoscope comprenant une tige allongée (2), un élément à ressort, un élément d'encliquetage à ressort de conception élastique pourvu d'un appui destiné à l'élément à ressort et d'un tube de système (11) qui s'étend à l'intérieur de la tige (2) et dans lequel est disposé un transmetteur d'image, le tube de système (11) étant maintenu dans une direction axiale de la tige (2) au moyen d'au moins ledit élément d'encliquetage à ressort,
**caractérisé en ce que** l'élément d'encliquetage à ressort, l'appui et l'élément à ressort sont conçus de telle sorte que le tube de système (11) soit contraint dans une direction distale contre une butée par l'élément à ressort.

2. Endoscope selon la revendication 1, **caractérisé en ce que** la butée est formée dans la région d'extrémité distale (4) de la tige (2).

3. Endoscope selon la revendication 2, **caractérisé en ce que** la butée est formée par un verre de recouvrement (32) qui est fixé dans la région d'extrémité distale de la tige (2) et contre lequel le tube de système (11) est en appui par le biais d'un objectif disposé entre le verre de recouvrement (32) et le transmetteur d'image (34).

4. Endoscope selon l'une des revendications précédentes, **caractérisé en ce que** l'endoscope (1, 1') comporte une tête d'endoscope (3) disposée à une extrémité proximale de la tige (2), **en ce que** le tube de système (11) s'étend jusque dans la tête d'endoscope (3) et **en ce que** l'élément d'encliquetage à ressort est reçu dans la tête d'endoscope (3) et relie une région d'extrémité proximale du tube de système (11) et un boîtier (7) de la tête d'endoscope (3).

5. Endoscope selon la revendication 4, **caractérisé en ce que** l'élément d'encliquetage à ressort est encliqueté dans au moins un évidement d'encliquetage formé sur un côté intérieur d'un manchon intérieur (21) de la tête d'endoscope (3).

6. Endoscope selon l'une des revendications précédentes, **caractérisé en ce que** le tube de système (11) est maintenu de manière amovible.

7. Endoscope selon l'une des revendications précédentes, **caractérisé en ce que** l'élément d'encliquetage à ressort est conçu sous la forme d'un manchon d'encliquetage (24) comprenant au moins un crochet d'encliquetage à ressort (25) disposé du côté extérieur.

8. Endoscope selon l'une des revendications précédentes, **caractérisé en ce que** l'élément d'encliquetage est conçu sous la forme d'une bague de serrage à ressort (40).

9. Endoscope selon l'une des revendications précédentes, **caractérisé en ce que** l'élément d'encliquetage comporte un ou plusieurs ergots d'encliquetage destinés à empêcher la rotation.

10. Endoscope selon l'une des revendications précédentes, **caractérisé en ce que** l'élément d'encliquetage à ressort comporte au moins un élément d'étanchéité circonférentiel et/ou au moins un évidement destiné à recevoir un agent dessicatif.

11. Procédé de fabrication d'un endoscope (1, 1') pourvu d'une tige allongée (2) et d'une tête d'endoscope (3), ledit procédé comprenant les étapes suivantes :
- fournir un boîtier d'endoscope qui comprend un tube extérieur (6) de la tige (2) et la tête d'endoscope (3), qui est reliée à celui-ci à une extrémité proximale de la tige (2) et qui comporte un manchon intérieur (21) qui comporte sur un côté intérieur au moins un évidement d'encliquetage,
- fournir un étage d'entrée de système qui comprend un tube de système (11) pourvu d'un transmetteur d'image reçu dans celui-ci et un élément d'accouplement (12) qui est relié de manière fixe à une extrémité proximale du tube de système (11),
- faire coulisser un ressort de compression (26) et un élément d'encliquetage à ressort sur l'élément d'accouplement (12) depuis une direction proximale,
- visser une monture d'oculaire (14) sur une extrémité proximale de l'élément d'accouplement (12),
- faire coulisser l'étage d'entrée de système dans le boîtier d'endoscope dans une direction distale jusqu'à ce qu'une butée soit atteinte et
- faire coulisser l'élément d'encliquetage sur l'élément d'accouplement (12) dans la direction distale jusqu'à ce que l'élément d'encliquetage s'engage dans l'au moins un évidement d'encliquetage.

12. Procédé selon la revendication 11, le boîtier d'endoscope comprenant un tube intérieur (28) disposé dans le tube extérieur (6) et relié de manière fixe au manchon intérieur (21) de la tête d'endoscope (3), lorsque l'étage d'entrée de système est coulissé jusque dans le boîtier d'endoscope, le tube de système (11) étant coulissé jusque dans le tube intérieur (28) et la butée étant formée dans la région d'extrémité distale du tube intérieur (28).

13. Procédé selon la revendication 11 ou 12, un oculaire étant inséré dans la monture d'oculaire (14) et ajusté après vissage de la monture d'oculaire (14) et avant coulissement de l'étage d'entrée de système jusque dans le boîtier d'endoscope.
